# EUROPEAN PATENT APPLICATION

(11) **EP 0 573 975 A1**
(43) Date of publication of application: **15.12.1993**
(21) Application number: 93109233.2
(22) Date of filing: 08.06.1993
(51) Int. Cl.: C07D 495/04, A61K 31/435

(54) **2-alkoxycarbonyl-5-o-chlorobenzyl-4,5,6,7-tetrahydrothieno[3,2-c]pyridine derivative and process for producing the same**

(30) Priority: 08.06.1992 JP 147189/92; 27.10.1992 JP 289041/92
(71) Applicant: FUJI PHOTO FILM CO., LTD., Kanagawa-ken (JP)
(72) Inventor: Yamakawa, Katsuyoshi, Minami-Ashigara-shi, Kanagawa-ken (JP); Sato, Kozo, Minami-Ashigara-shi, Kanagawa-ken (JP); Suginome, Takashi, Odawara-shi, Kanagawa-ken (JP)
(74) Representative: Hansen, Bernd, Dr. Dipl.-Chem.

(57) **Abstract**

An intermediate, 2-alkoxycarbonyl-5-o-chlorobenzyl-4,5,6,7-tetrahydrothieno[3,2-c]pyridine-2-carboxylic acid derivatives of the following general formula (I): 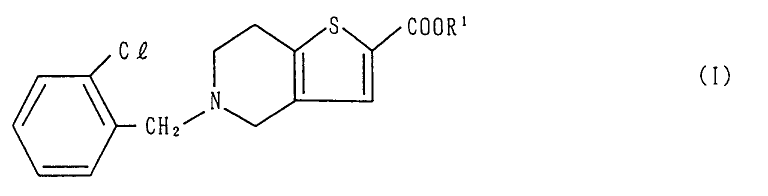
wherein R¹ represents an alkyl group
and acid salts of them are useful for synthesizing ticlopidine hydrochloride from an inexpensive, easily available compound in short steps.

## Description

### Background of the Invention

The present invention relates to new synthetic intermediates useful for producing ticlopidine hydrochloride having an effect of inhibiting platelet aggregation in an economical manner on an industrial scale, and a process for producing them.

Reports were made from old times on the synthesis of a 4,5,6,7-tetrahydrothieno[3,2-c]pyridine skeleton of ticlopidine hydrochloride. The processes for the synthesis can be roughly divided into two processes. One is a process wherein a thiophene derivative is used as the starting material and a tetrahydropyridine ring is closed [see, for example, Japanese Patent Publication for Opposition Purpose (hereinafter referred to as "J.P. KOKOHU") No. Sho 56-2068, Japanese Patent Unexamined Published Application (hereinafter referred to as "J.P. KOKAI") No. Sho 62-103088 and EP 439404A2] and the other is a process wherein a piperidone derivative is used as the starting material and a thiophene ring is closed (see, for example, J.P. KOKAI Nos. Sho 63-2992 and Sho 63-126883, EP 360293A2 and DE 2,701,511). Although the former (known reaction scheme 1) comprising only a small number of the reaction steps is an advantageous process, a further improvement is demanded, since it has problems that a cyanide is used as the starting material and that side reactions occur in the course of the reduction. The investigations of the latter (known reaction schemes 2, 3 and 4) were started relatively recently and only a very small number of reports were proposed. Thus no process for producing the intended compound from an inexpensive starting material by short steps has been found yet.
Known reaction scheme 1 (J.P. KOKAI No. Sho 62-103088 etc.) 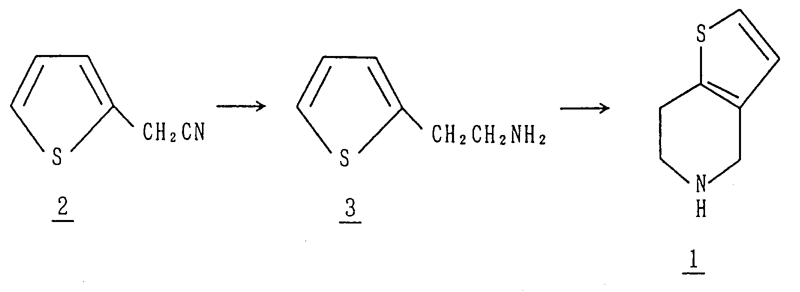
Known reaction scheme 2 (EP 360293A2) 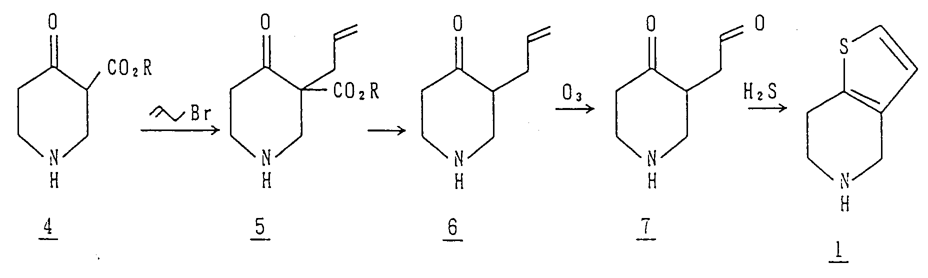
Known reaction scheme 3 (J.P. KOKAI No. Sho 63-2992) 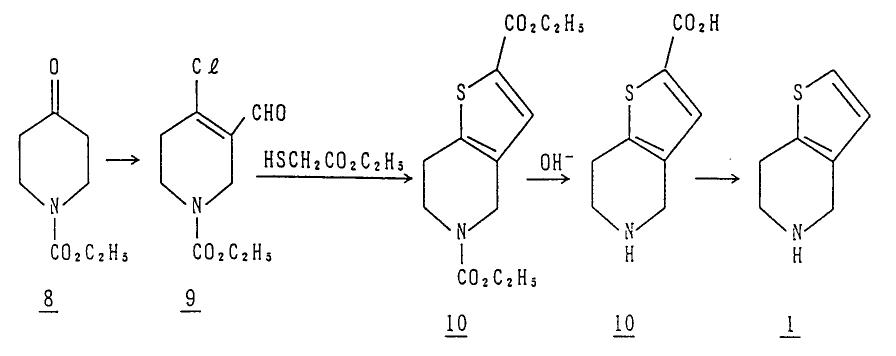
Known reaction scheme 4 (J.P. KOKAI No. Sho 63-126883)

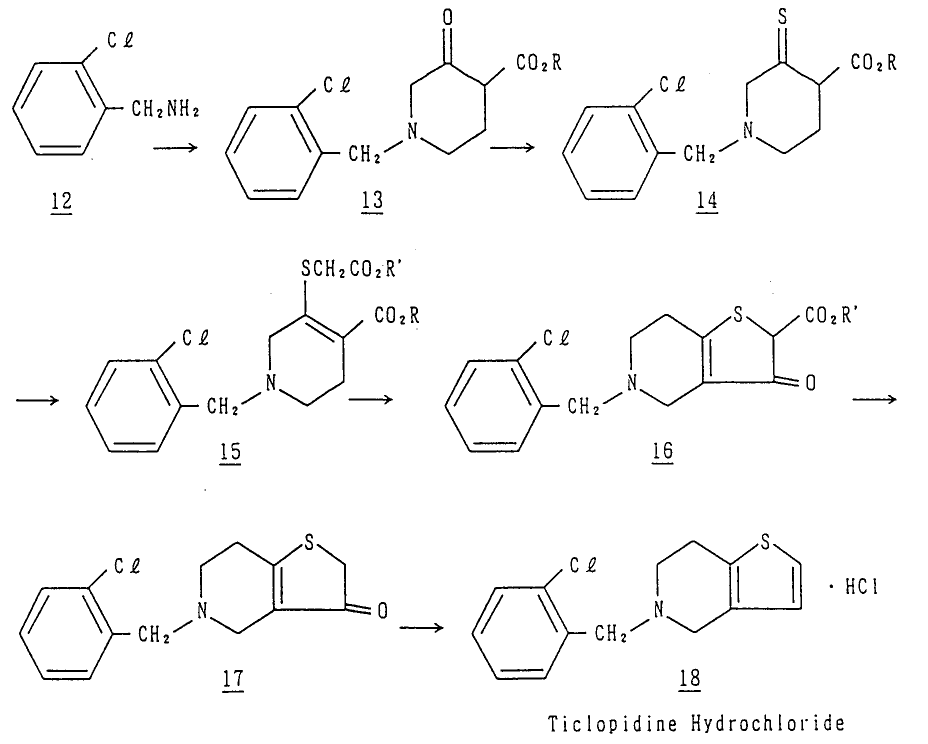

### Summary of the Invention

The object of the present invention is to provide an intermediate useful for synthesizing ticlopidine hydrochloride from an inexpensive, easily available compound in short steps.

Another object of the present invention is to provide a process for producing an intermediate useful for synthesizing ticlopidine hydrochloride from an inexpensive, easily available compound in short steps.

These and other objects of the present invention will be apparent from the following description and Examples.

The first aspect of the invention relates to 2-alkoxycarbonyl-5-o-chlorobenzyl-4,5,6,7-tetrahydrothieno[3,2-c]pyridine-2-carboxylic acid derivatives of the following general formula (I): 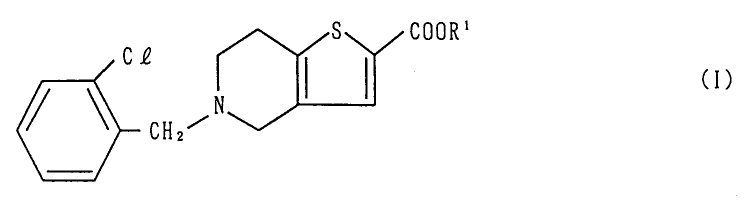
wherein R¹ represents an alkyl group
and acid salts of them.

The second aspect of the invention relates to a process for producing a 2-alkoxycarbonyl-5-o-chlorobenzyl-4,5,6,7-tetrahydrothieno[3,2-c]pyridine derivative of the general formula (I), which comprises diazotizing a 2-alkoxycarbonyl-3-amino-5-o-chlorobenzyl-4,5,6,7-tetrahydrothieno[3,2-c]pyridine derivative of the general formula (II): 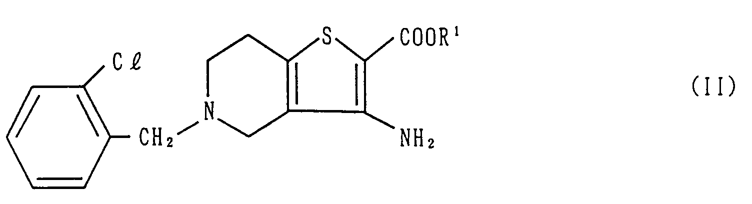
wherein R¹ is as defined above.
and then subjecting the thus-obtained diazonium salt to a reductive nitrogen-removing reaction.

### Detailed explanation of Preferred Embodiments

The derivatives of the present invention are novel and the derivatives per se are expected to have physiological acivities such as anti-thrombogenic acivity and the like.

The derivatives of the general formula (I) are synthetic intermediates useful for producing ticlopidine hydrochloride by the following reaction scheme: 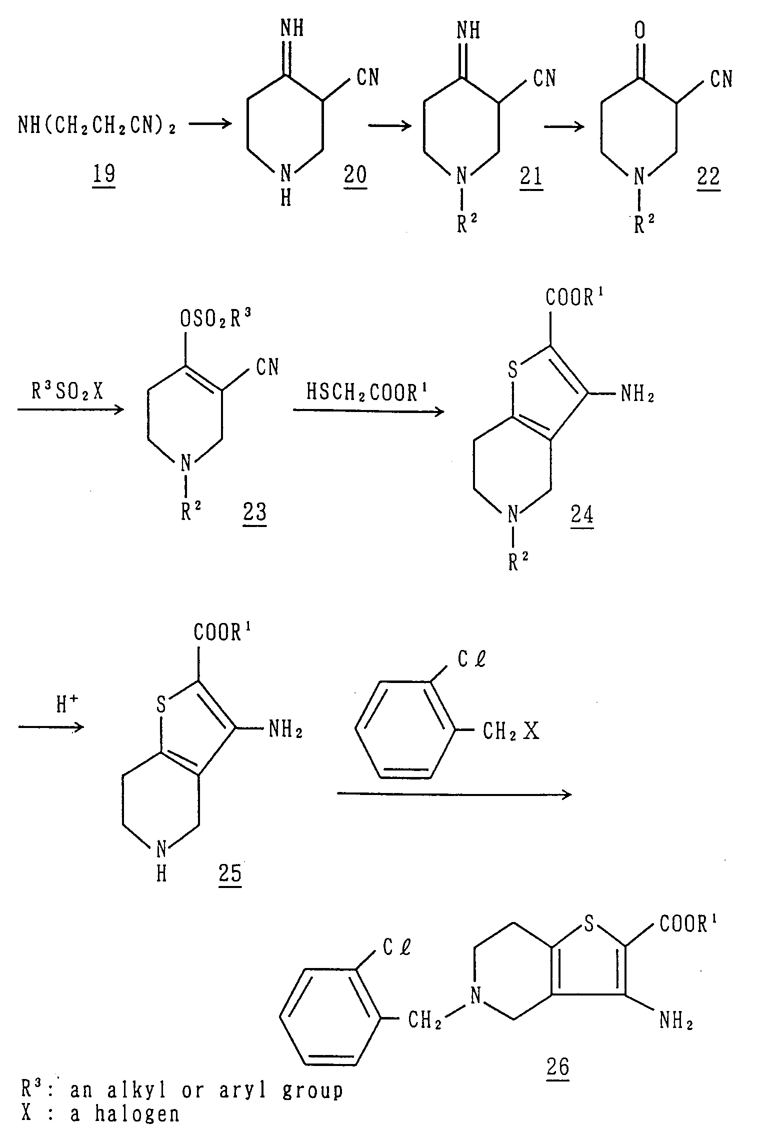 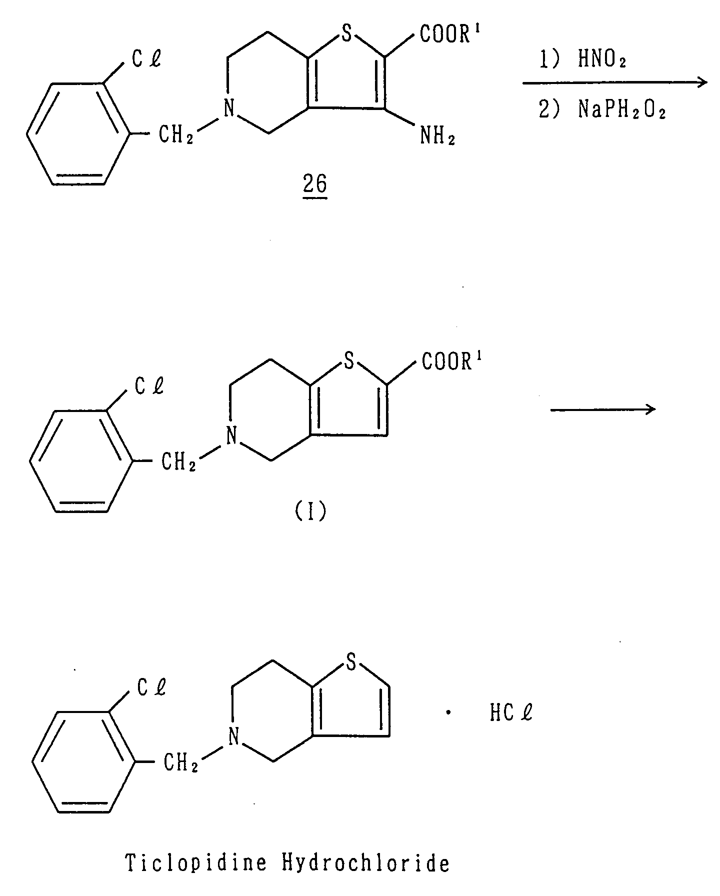
The detailed description will be made on the compounds of the present invention hereafter.

R¹ in the above general formula (I) represents alkyl groups having preferably 1 to 20 carbon atoms, more preferably 1 to 8 carbon atoms (such as methyl, ethyl, butyl and 2-ethylhexyl groups).

The acids capable of forming the acid salt with the compound of the general formula (I) include inorganic acids such as hydrochloric acid, sulfuric acid, hydrobromic acid, etc.; and organic acids such as acetic acid, p-toluenesulfonic acid, etc. The most preferred salt is hydrochloride. The acid salt of the compound of the general formula (I) may be prepared by adding the acid according to the known method.

Examples of the compounds of the general formula (I) of the present invention are listed in Table 1.

**Table 1**

| Compound No. | R¹ |
|---|---|
| (I)-1 | CH₃ |
| (I)-2 | C₂H₅ |
| (I)-3 | C₃H₇ |
| (I)-4 | C₄H₉ |
| (I)-5 | C₈H₁₇ |
| (I)-6 | C₄H₉CH(C₂H₅)CH₂ |
| (I)-7 | C₁₂H₂₅ |
| (I)-8 | C₁₄H₂₉ |
| (I)-9 | C₈H₁₇CH(C₆H₁₃)CH₂ |
| (I)-10 | C₁₈H₃₇ |

The detailed description will be made on the diazotization and reduction reactions to obtain the compound of the general formula (I), referring to the above reaction scheme.

Compound 26 (derivative of the general formula (II)) is synthesized from 3,3'-iminodipropionitrile l9 which is the starting material and easily available on the market at a low cost (Japanese Patent Application No. Hei 4-147185). This compound is deanimated through a diazotization and reduction reactions to obtain a compound of the general formula (I) in a high yield.

R² of the compounds 21 to 24 represents an alkylcarbonyl group, arylcarbonyl group, alkylsulfonyl group, arylsulfonyl group, alkoxycarbonyl group or aryloxycarbonyl group and the like, preferably acetyl group. Ticlopidine hydrochloride is produced through a hydrolysis, decarboxylation and the like of the compound of the general formula (I).

The nitrites usable for forming the diazonium compounds include alkali nitrites such as sodium nitrite, potassium nitrite and lithium nitrite. In them, sodium nitrite is particularly preferred. The acids usable for the diazonium-forming reaction are not particularly limited so far as they are capable of liberating nitrous acid from the nitrite. They include, for example, hydrochloric acid, sulfuric acid, hydrobromic acid and acetic acid. In them, hydrochloric acid is particularly preferred. The solvents include water, alcohols, THF, etc. They can be used either singly or in the form of a mixture of them. Particularly preferred solvent is water. The amount of the nitrite used is usually 0.8 to 1.2 mol, preferably 0.9 to 1.1 mol, per mol of the compound of the general formula (II), and the amount of the acid used is 2.0 to 4.0 mol, preferably 2.5 to 3.0 mol, per mol of the nitrite. When excess nitric acid remains in the reaction liquid after completion of the reaction, it can be decomposed by addition of urea. The reaction temperature is usally -5 to 10°C , preferably 0 to 5°C .
The reaction time which markedly varies depending on the kinds and relative amounts of the nitrite, acid used and the reaction temperature is usually 0.5 to 5 hours, preferably l to 2 hours. After completion of the reaction, the diazonium salt can be isolated. Usually, however, the reaction product is subjected to the subsequent reaction without separation.

Hypophosphorous acid or ethanol is preferably used as the reducing agent for the nitrogen-removing reaction of the diazonium salt obtained as described above since it is inexpensive. Further tributyltin hydride, triethylsilane hydride, sodium borohydride, etc. are also usable. The solvent used for forming the diazonium salt can be used as it is for this reaction. When tributyltin hydride or the like is used, an organic solvent such as diethyl ether, THF or acetonitrile can be used. The amount of the reducing agent is l to l00 mol, preferably l to l0 mol, per mol of the diazonium salt. The reaction temperature is usually -20 to +20 °C , preferably -10 to +10 °C . The reaction time which markedly varies depending on the variety and amount of the reducing agent used and reaction temperature is usually 0.5 to 3 hours, preferably 0.5 to 2 hours. After completion of the reaction, the intended product can be isolated by an ordinary method such as neutralization, extrusion, extraction, washing, concentration or crystallization. The product can be purified, if necessary, by recrystallization, column chromatography or the like.

Ticlopidine hydrochloride can be easily obtained by subjecting the compound of the general formula (I) to either the hydrolysis and decarboxylation at the same time in the presence of an acid such as hydrobromic acid, or decarboxylation with copper/quinoline after completion of the hydrolysis and isolation of the hydrolyzed compound, or a dealkoxycarboxylation in the presence of lithium iodide-collidine(quinoline) or copper iodide-collidine(quinoline).

The present invention provides a new 2-alkoxycarbonyl-5-o-chlorobenzyl-4,5,6,7-tetrahydrothieno[3,2-c]pyridine derivative and salt thereof useful for producing ticlopidine hydrochloride having an effect of inhibiting platelet aggregation in an economical manner on an industrial scale, and an advantageous process for producing them.

The following Examples are given to illustrate the invention.

### (Examples)

Examples for synthesises of representative compounds of the present invention and analytical data thereof will be shown below.

### Example 1 Synthesis of conpound (I)-1

Conpound (I)-1 was synthesized according to the following reaction scheme: 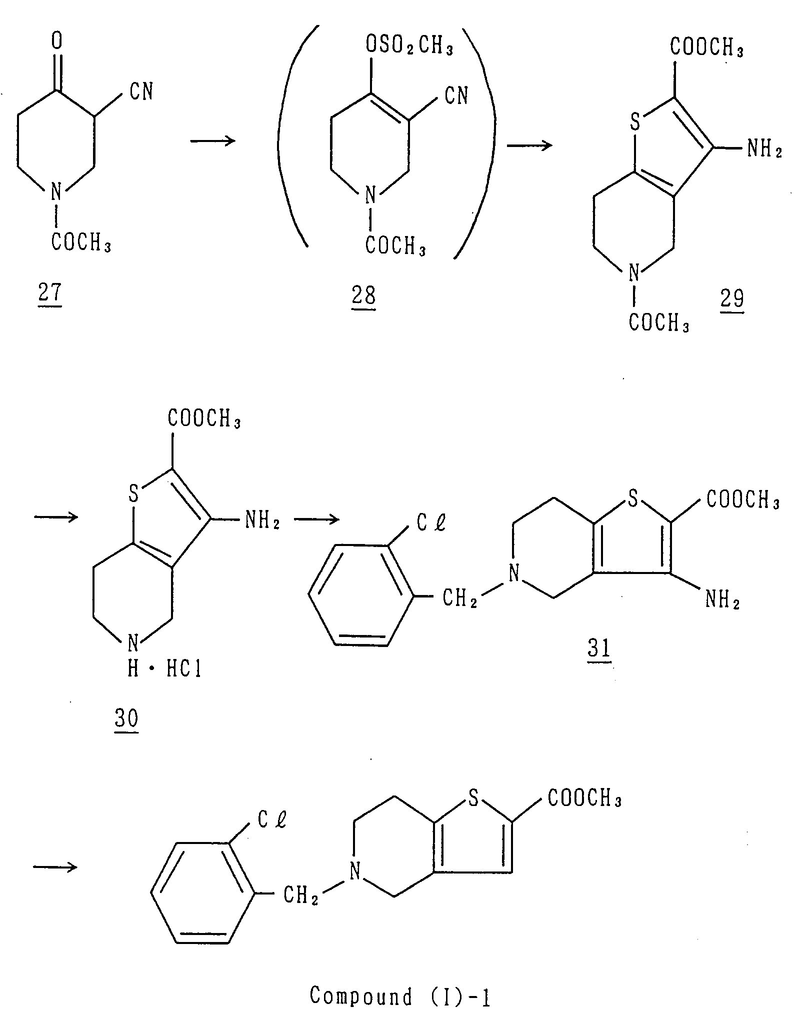
8.3 g (0.05 mol) of l-acetyl-3-cyanopiperidine-4-one (27) which is a compound known from a literature was dissolved in ll.9 g (0.l5 mol) of pyridine. 5.7 g (0.05 mol) of methanesulfonyl chloride was added dropwise to the resultant solution under cooling with ice and then the resultant mixture was stirred at room temperature for 30 min. Then 5.4 g (0.05 mol) of methyl thioglycolate was mixed with l9.3 g (0.l mol CH₃ONa) of 28 % solution of sodium methylate in methanol. The resultant mixture was stirred for 5 min and then added to the reaction solution obtained as described above. The resultant mixture was stirred at room temperature for 30 min and then cooled with ice. 9.6 g (0.05 mol CH₃ONa) of 28 % solution of sodium methylate in methanol was added to the reaction solution and the resultant mixture was stirred at room temperature for 30 min.

After completion of the reaction, 800 ml of water was added to the reaction solution. After neutralization (pH 5 to 6) with hydrochloric acid followed by extraction with ethyl acetate (600 ml × 2 ), the ethyl acetate layer was washed with 500 ml of 3 N aqueous sodium hydroxide solution and then with 500 ml of water. Ethyl acetate was distilled off under reduced pressure and the residue was recrystallised from acetonitrile to obtain 5.8 g (46 %) of methyl 5-acetyl-3-amino-4,5,6,7-tetrahydrothieno[3,2-c]pyridine-2-carboxylate (29).

To 2.54 g of compound 29, 20 ml of methanol and 5.1 ml of concentrated hydrochloric acid were added in this order and then the reaction mixture was heated under reflux for 4 hours. After cooling, the crystals thus precipitated were collected by filtration to obtain l.70 g of methyl 3-amino-4,5,6,7-tetrahydrothieno[3,2-c]pyridine-2-carboxylate hydrochloride (30). Yield: 68 %.
Melting point: 270°C or above
¹H-NMR(200MHz)
δ ppm ( DMSO-d₆): 2.95(2H, t, J=6.0 Hz), 3.40(2H, t, J=6.0 Hz) , 3.71(3H, s), 3.95(2H, t), 6.60(2H, s), 9.76(2H, s).

Subsequently, 2.48 g of compound 30 and l.6l g of o-chlorobenzyl chloride were dispersed in 20 ml of acetonitrile. 3.1 ml of triethylamine was added to the dispersion and the resultant mixture was heated under reflux for 4 hours. After cooling, ethyl acetate and water were added thereto. After fractionation followed by washing with water, the product was dried over Glauber's salt. The solvent was distilled off and the residue was purified by column chromatography (n-hexane / ethyl acetate = 1/1). The crystals were formed with a solvent mixture of n-hexane / ethyl acetate to obtain l.42 g (42 %) of methyl 3-amino-5-o-chlorobenzyl-4,5,6,7-tetrahydrothieno[3,2-c]pyridine-2-carboxylate (31).
Melting point: 120-121°C
¹H-NMR(200MHz)
δ ppm ( DMSO- d₆): 2.75(bs, 2H), 3.35(s, 2H), 3.69(s, 3H), 3.77(s, 2H), 6.38(s, 2H), 7.32(m, 4H).

Subsequently, to 3.3 g of compound 31, 3 ml of water and 30 ml of sulfuric acid were added in this order. Then, a solution of 4.2 g of sodium nitrite in 18 ml of water was added dropwise little by little to the reaction mixture at a temperature of 0 to 5°C under cooling with freezing mixture (ice-methanol) and then the reaction mixture was stirred for 30 minutes. To this, 50 ml of hypophosphorous acid which had been preliminarily cooled with ice was added little by little. After stirring for 1 hour at room temperature, the resultant mixture was poured into 300 ml of water and then neutralized (pH 6) with aqueous solution of sodium hydroxide. The resultant mixture was satulated with salt and then extracted with ethyl acetate (300 ml × 1). The ethy l acetate layer was washed with satulated aqueous solution of salt (200 ml× 2) and then ethyl acetate was distilled off under reduce d pressure. The residue was purified by column chromatography (n-hexane / ethyl acetate = 5/1) to obtain l.6 g (50 %) of compound (I)-1 as an oily substance.
¹H-NMR(200MHz)
δ ppm (CDCl₃): 2.87(m, 4H), 3.59(s, 2H), 3.80(m, 2H), 3.85(s, 3H), 7.21(m, 2H), 7.37(m, 1H), 7.43(s, 1H), 7.50(m, 1H).

Other compounds of the present invention can be obtained in the same manner as described above.

### Example 2 Compound (I)-4

The following is analytical data of compound (I)-4 synthesized in the same manner as that of Example 1.
Colorless cristal
Melting point: 53-55°C (crystalization solvent: methanol)
¹H-NMR(200MHz)
δ ppm (CDCl₃): 0.95(t, 3H, J=7.0Hz), 1.43(tq, 2H, J=7.0, 7.0Hz), 1.67(tt, 2H, J=7.0, 7.0Hz), 2.87(m, 4H), 3.61(s, 2H), 3.80(s, 2H), 4.25(t, 2H, J=7.0Hz), 7.22(m, 2H), 7.39(dd, 1H, J=8.7, 3.3Hz), 7.41(s, 1H), 7.51(dd, 1H, J=8.0, 3.0Hz)

### Example 3 Compound (I)-6

The following is analytical data of compound (I)-6 synthesized in the same manner as that of Example 1.
Oil
¹H-NMR(200MHz)
δ ppm (CDCl₃ ): 0.85(t, 3H, J=7.0Hz), 0.87(t, 3H, J=7.0Hz), 1.28(m, 4H), 1.36(q, 2H, J=7.0Hz), 1.39(q, 2H, J=7.0Hz), 1.63(m, 1H), 2.85(m, 4H), 3.60(s, 2H), 3.81(s, 2H), 4.15(d, 2H, J=6.0Hz), 7.23(m, 2H), 7.36(dd, 1H, J=9.3, 2.7Hz), 7.38(s, 1H), 7.50(dd, 1H, J=7.2, 2.3Hz)

### Example 4 Compound (I)-9

The following is analytical data of compound (I)-9 synthesized in the same manner as that of Example 1.
Oil
¹H-NMR(200MHz)
δ ppm (CDCl₃ ): 0.86(t, 6H, J=6.7Hz), 1.24(m, 24H), 1.71(m, 1H), 2.88(m, 4H), 3.60(s, 2H), 3.79(s, 2H), 4.14(d, 2H, J=6.0Hz), 7.23(m, 2H), 7.36(dd, 1H, J=8.6, 2.0Hz), 7.39(s, 1H), 7.50(dd, 1H, J=6.7, 2.0Hz)

## Claims

1. A compound of the general formula (I): 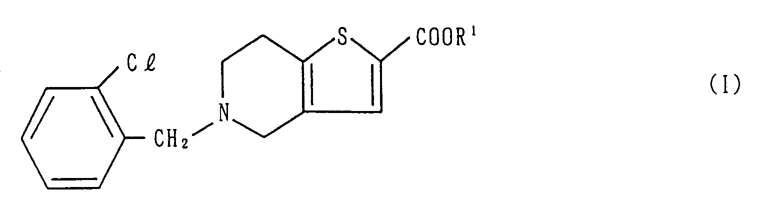 wherein R¹ represents an alkyl group
and acid salts thereof.

2. The compound and acid salt thereof according to claim 1 wherein R¹ is an alkyl group having 1 to 20 carbon atoms.

3. The compound according to claim 2 wherein R¹ is an alkyl group having l to 8 carbon atoms.

4. A process for producing a compound of the general formula (I) as defined in claim 1, which comprises diazotizing a compound of the general formula (II): 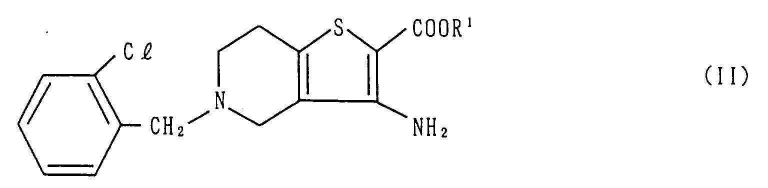 wherein R¹ represents an alkyl group
and then subjecting the thus-obtained diazonium salt to a reductive nitrogen-removing reaction.

5. The process according to claim 4 wherein R¹ is an alkyl group having 1 to 20 carbon atoms.

6. The process according to claim 5 wherein R¹ is an alkyl group having l to 8 carbon atoms.

7. The process according to claim 4 wherein the amount of the nitrite is 0.8 to 1.2 mol per mol of the compound of the general formula (II).

8. The process according to claim 4 wherein the acid used for the diazonium-forming reaction is hydrochloric acid, sulfuric acid, hydrobromic acid or acetic acid and the amount of the acid is 2.0 to 4.0 mol per mol of the nitrite.

9. The process according to claim 4 wherein the reductive nitrogen-removing reaction is carried out using hypophosphorous acid or ethanol as a reducing agent.

10. The process according to claim 6 wherein the amount of the nitrite is 0.8 to 1.2 mol per mol of the compound of the general formula (II), the acid used for the diasonium-forming reaction is hydrochloric acid, sulfuric acid, hydrobromic acid or acetic acid, the amount of the acid is 2.0 to 4.0 mol per mol of the nitrite, and the reductive nitrogen-removing reaction is carried out using hypophosphorous acid or ethanol as a reducing agent.

11. Use of a compound of the general formula (I) as defined in claim 1 for a pharmaceutical composition for treatment of thrombogenesis.
